# FASCICULE DE BREVET EUROPEEN

(11) **EP 1 460 124 B1**
(45) Date de publication et mention de la délivrance du brevet: **11.07.2012**
(21) Numéro de dépôt: 04290455.7
(22) Date de dépôt: 20.02.2004
(51) Int. Cl.: C11C 3/10, C07C 67/03, C07C 69/52, C10L 1/02

(54) **Procédé d'alcoolyse d'huiles acides d'origine vegetale ou animale**
Verfahren zur Alkoholyse von pflanzlichen oder tierischen sauren Ölen
Process for alcoholysis of vegetable or animal acid oils

(30) Priorité: 17.03.2003 FR 0303575
(43) Date de publication de la demande: 22.09.2004
(73) Titulaire: Institut Français du Pétrole, 92852 Rueil-Malmaison Cedex (FR)
(72) Inventeur: Hillion, Gérard, 95220 Herblay (FR); Le Pennec, Dominique, 78910 Orgerus (FR)

(56) Documents cités:
- EP-A- 0 924 185
- EP-A- 1 352 893
- WO-A-93/01263
- US-A- 5 908 946

## Description

La présente invention concerne l'alcoolyse d'huiles acides non désacifiées d'origine végétale ou animale.

Elle a plus particulièrement pour objet un procédé consistant à utiliser une huile de départ d'origine végétale ou animale possédant une acidité grasse libre, exprimée par son indice d'acide (I.A.). à la transestérifier par un monoalcool de C1 à C6 en estérifiant simultanément l'acidité libre, ce par un monoalcool de C1 à C6 pour produire des esters de monoalcools de C1 à C6 et d'acides gras de C6 à C26.

Dans la présente invention, l'indice d'acide (I.A.) est compris entre 0,5 et 20, par exemple entre 1 et 15 et de préférence compris entre 2 et 12.

L'I.A. s'exprime par le nombre de mg de KOH nécessaire pour neutraliser 1 g de produit. L'acidité d'une huile ou d'une matière grasse, peut également être exprimée en % masse d'acide gras. C'est généralement l'acide gras dont la teneur est majoritaire dans l'huile qui est pris en compte, par exemple pour une huile de colza, l'acidité sera exprimée en % masse d'acide oléique.

Contrairement aux procédés d'estérification d'huiles végétales utilisant la catalyse basique homogène, on a découvert de façon surprenante qu'en utilisant par exemple une matière grasse possédant un indice d'acide compris entre 0,5 et 20, par exemple entre 1 et 15 et plus particulièrement entre 2 et 12, on pouvait réaliser simultanément l'estérification des acides gras et la transestérification de l'huile par le méthanol en utilisant dans certaines conditions un catalyseur hétérogène en lit fixe. Cette réaction peut s'étendre aux monoalcools supérieurs comme l'éthanol, le propanol-1, l'isopropanol, le butanol-1, l'isobutanol, le pentanol-1 ou l'hexanol-1.

Ce procédé catalytique utilise deux étapes réactionnelles et permet de transformer simultanément les triglycérides et les acides gras en esters méthyliques, par exemple dans le cadre d'une production d'esters à usage carburants.

On peut également utiliser les propriétés catalytiques de ce système pour transestérifier les esters de monoalcools obtenus par des alcools lourds comme le 2-éthylhexanol ou par des polyols comme par exemple, le propylèneglycol 1-2 , le propylèneglycol 1-3, le glycérol, le néopentylglycol, le triméthylolpropane, le pentaérythritol, etc. Dans ces conditions, on travaille à pression atmosphérique de façon à favoriser le départ du monoalcool du milieu réactionnel, ce qui permet de déplacer fortement l'équilibre de réaction.

Dans les procédés classiques de production d'esters qui utilisent la catalyse basique (hydroxydes ou alcoolates alcalins), l'indice d'acide des huiles utilisées est dans la majorité des cas inférieur à 1. Pour des valeurs supérieures à 1, la catalyse basique homogène devient rédhibitoire, car la quantité de catalyseur à utiliser devient alors directement proportionnelle à celle de l'indice d'acide de l'huile utilisée. Ceci conduit à la formation d'une quantité importante de savons, ce qui diminue d'autant le rendement global de l'opération, sans compter les étapes de traitements délicats et coûteux d'élimination des savons fabriqués.

Par exemple, pour effectuer la méthanolyse d'une huile végétale ou animale neutralisée dont l'Indice d'Acide est ≤ 0,5, la quantité de méthylate de sodium nécessaire pour obtenir une conversion optimale en esters méthyliques est de l'ordre de 70 moles en équivalent sodium/tonne d'huile mise en jeu. Dans le cas d'une huile possédant un I.A. égal à 10, la quantité d'alcoolate à mettre en jeu serait alors augmentée de 180 moles en équivalent sodium et représenterait une quantité supplémentaire de savons de l'ordre de 55 kg / tonne d'huile. Outre le surcoût en catalyseur, l'élimination d'une telle quantité de savons reste délicate et coûteuse, engendrant une baisse de rendement de l'ordre de 5,5 % en masse.

C'est pourquoi il est indispensable de recourir à un raffinage de l'huile de façon à réduire l'acidité libre, soit par un traitement chimique soit par un traitement physique selon le degré d'acidité initial de l'huile utilisée.

Le document US 2002/0352802 décrit un procédé d'alcoolyse de triglycérides d'acides gras d'origine végétale ou animale, utilisant un catalyseur à base de carbonate de calcium. Si les triglycérides contiennent des acides gras libres, il est nécessaire de les estérifier préalablement avec un catalyseur acide, puis de les neutraliser avec un lit de carbonate de calcium.

La présente invention concerne l'utilisation de toutes huiles végétales brutes débarrassées de leurs phospholipides ou mucilages, les huiles de récupération comme celles utilisées en fritures, ainsi que les graisses animales dont les indices d'acide (I.A.) mesurés sont compris entre 0,5 et 20.

On peut citer par exemple, comme procédés conventionnels de raffinage, la distillation neutralisante applicable aux huiles concrètes, comme le palme, le palmiste et le coprah, ces dernières ne contenant pas ou très peu de phospholipides et qui consiste en un entraînement à la vapeur d'eau sous vide poussé par exemple à une température de 235 °C.

Pour les autres types d'huiles comme le soja, colza, tournesol, maïs; arachide, coton, karité, crambe, carthame, ricin, etc., c'est le raffinage chimique qui est réalisé après une étape de démucilagination ou de dégommage consistant à rendre insoluble les phospholipides par hydratation.

L'élimination de ces phospholipides hydratés peut être réalisée :
- séparément avant l'étape de neutralisation, par exemple dans le cas du soja où ces produits (les phospholipides), riches en lécithine, sont valorisés dans l'industrie alimentaire comme agents émulsifiants ;
- ou en même temps que les savons après l'étape de neutralisation à la lessive de soude.

La séparation des savons résultant de l'étape de neutralisation par une solution de lessive de soude est dans la plupart des cas réalisée par centrifugation. Les savons de sodium obtenus sont également appelés "soapstocks" ou pâtes de neutralisation. Cela représente la plus grande partie des pertes en matières grasses dans le raffinage de l'huile. Le taux d'imprégnation de l'huile dans les pâtes de neutralisation varie de 15 à 100 %, ceci pouvant être exprimé par le coefficient de neutralisation, qui, dans ce cas, est compris entre 1,15 et 2.

Cette perte en matière grasse est proportionnelle à l'indice d'acide de l'huile et peut atteindre de 2 à 4 % en masse de l'huile mise en jeu pour les huiles européennes courantes, comme par exemple l'huile de colza ou l'huile de tournesol.

Cette fraction constituée des pâtes de neutralisation est faiblement valorisée sous la forme d'une huile acide obtenue suite à une étape de neutralisation par un acide fort, qui est appelée le "cassage" des pâtes de neutralisation.

Certains procédés de production de "Biodiesel" qui utilisent des huiles acides comme par exemple l'huile de palme intègrent une pré-étape qui consiste à estérifier les acides gras libres par un monoalcool de C1 à C5 en présence d'un catalyseur souvent à caractère fortement acide comme l'acide sulfurique (brevet US-A-2 383 601) ou chlorhydrique, ou un acide sulfonique soluble ou supporté sous la forme de résines échangeuses d'ions recyclables (brevet français FR-B-2 577 938).

La réduction de l'acidité devant permettre de réaliser l'étape suivante qui consiste à transestérifier par catalyse basique (alcoolate alcalin) la totalité de l'huile.

Ces procédés, qui utilisent souvent le méthanol, demandent des temps de séjour assez long et des quantités importantes d'alcool. Cet excès d'alcool est nécessaire pour déplacer l'équilibre en éliminant l'eau formée au cours de la réaction d'estérification. C'est un entraînement physique de l'eau qui est réalisé, car le mélange méthanol/eau ne forme pas d'azéotrope. Une rectification du mélange méthanol/eau récupéré est dans ce cas nécessaire de façon à recycler ce large excès d'alcool.

Une autre solution envisagée pour l'utilisation d'huiles fortement acides, cas particulier des huiles de palme qui peuvent avoir des I.A. > 30, est de réduire cette acidité palmitique en effectuant une étape de glycérolyse des acides gras libres, qui consiste à estérifier avec une faible quantité de glycérol les acides gras libres, en utilisant le même catalyseur que celui décrit dans l'invention. Ce procédé étant particulièrement adapté à la production d'esters carburants comme décrit dans le brevet US-A-5 908 946, déposé par la demanderesse, qui décrit également le mode de préparation du catalyseur ainsi que les conditions de catalyse.

L'invention vise donc à supprimer certains traitements associés aux étapes du raffinage des huiles végétales, qui habituellement réduisent leur indice d'acide à des valeurs basses, souvent inférieures à 1, ce qui revient à utiliser dans le procédé de l'invention une huile végétale non désacidifiée possédant un indice d'acide final compris entre 0,5 et 20, par exemple entre 1 et 15 et de préférence entre 2 et 12, qui est issue de pression et/ou d'extraction et qui a subi une étape de démucilagination, de façon à obtenir une teneur résiduelle en phosphore inférieure à 10 ppm, suivie d'une étape de séchage permettant d'obtenir une teneur en eau résiduelle inférieure à 500 ppm.

L'invention propose un procédé d'alcoolyse d'huiles acides non désacidifiées d'origine végétale ou animale permettant, avec des monoalcools de C1 à C5, de transestérifier des huiles végétales ou animales possédant une acidité libre naturelle, et d'estérifier simultanément leur acidité libre, caractérisé en ce qu'il comprend deux étapes de catalyse dans deux réacteurs fonctionnant avec un catalyseur hétérogène en lit fixe.

Le procédé de l'invention peut être défini plus particulièrement par le fait qu'il comprend :
- une étape (a) de réaction catalytique, dans laquelle on introduit simultanément, une quantité aliquote d'huile et de monoalcool dans un premier réacteur, préchauffé à une température comprise entre 180 et de 210°C et sous une pression opératoire comprise entre 40 et 60 bar (4 à 6 MPa) ;
- une étape (b) dans laquelle on soumet le mélange réactionnel en sortie dudit premier réacteur de catalyse à une évaporation totale ou partielle de l'excès de monoalcool, favorisant la séparation du glycérol formé, que l'on récupère ;
- une étape (c) dans laquelle on introduit le mélange d'ester dans un deuxième réacteur avec addition de l'équivalent massique de monoalcool ;
- une étape (d) dans laquelle on soumet le mélange issu de l'étape (c) à une évaporation totale de l'excès de monoalcool, puis l'élimination du glycérol résiduel formé.

De préférence dans le procédé de l'invention, les deux réacteurs sont de tailles substantiellement identiques et l'étape (c) est effectuée dans les mêmes conditions de catalyse que la première étape de catalyse (a).

Le procédé de l'invention consiste donc à travailler en deux étapes de catalyse dans deux réacteurs de tailles substantiellement identiques et qui fonctionnent avec un catalyseur hétérogène en lit fixe.

Le catalyseur hétérogène comprend en général un aluminate de zinc et répond à la formule :

ZnAl₂O₄, x ZnO, y Al₂O₃

x et y étant compris entre 0 et 2, l'aluminate de zinc ayant plus particulièrement une structure de type spinelle.

On introduit simultanément, par l'intermédiaire de deux pompes doseuses, une quantité aliquote d'huile et de monoalcool dans le premier réacteur, qui est préchauffé à une température comprise entre 180 et de 210 °C et sous une pression opératoire comprise entre 40 et 60 bar (4 à 6 MPa). En sortie du premier réacteur de catalyse, le mélange réactionnel est soumis à une évaporation totale ou partielle de l'excès de méthanol, favorisant la séparation du glycérol formé, qui est récupéré après une étape de décantation statique.

Cette étape intermédiaire d'élimination du glycérol permet de déplacer l'équilibre de la réaction afin d'obtenir, dans le second réacteur, une conversion maximale en esters méthyliques.

Pour ce faire, le mélange d'ester ainsi obtenu est ensuite introduit dans le deuxième réacteur avec addition de l'équivalent massique de méthanol. Les conditions de catalyse sont identiques à celles préconisées dans la première étape de catalyse.

A l'issue de cette deuxième étape, le mélange subit une évaporation totale de l'excès de méthanol, puis l'élimination du glycérol résiduel formé.

Le produit fini, qui est caractérisé par un mélange d'esters d'acides gras, répond aux spécifications en vigueur en tant que carburant pour moteurs Diesel, tant sur la teneur en esters méthyliques que sur la valeur de son indice d'acide final.

Les exemples présentés ci-après ne limitent pas l'invention et ne servent qu'à l'illustrer.

### EXEMPLE 1

On réalise dans un réacteur en continu en présence d'un catalyseur hétérogène et fonctionnant en lit fixe une transestérification par le méthanol d'une huile de colza semi-raffinée (l'huile de colza semi-raffinée n'a pas subi les étapes de décoloration et de désodorisation nécessaire pour obtenir la qualité d'huile alimentaire).

Cela consiste à faire passer dans un réacteur mono-tubulaire contenant 70 ml de catalyseur à base d'extrudés d'aluminate de zinc un mélange 50/50 en masse d'huile et de méthanol introduit séparément par l'intermédiaire de deux pompes doseuses. Les volumes correspondant étant de 35 ml d'huile et 40 ml de méthanol /heure, correspondant à un temps de séjour du mélange huile /méthanol sur le catalyseur d'environ 56 minutes.

La température est maintenue à 200 °C et la pression stabilisée entre 50 et 60 bar (entre 5 et 6 MPa).

Après un temps de fonctionnement de 4 à 5 heures permettant d'obtenir une conversion stabilisée, l'analyse du mélange réactionnel par GPC (Gel Permeation Chromatography) permet de quantifier la composition du mélange, qui est constitué principalement d'esters méthyliques, de triglycérides non convertis, de diglycérides, de monoglycérides et de stérols libres ou partiellement estérifiés.

On laisse fonctionner ce procédé pendant au moins 72 heures de façon à recueillir une quantité suffisante de produits, soit environ 2,5 litres de mélange d'esters, qui serviront à alimenter le même réacteur de façon à simuler une deuxième étape de catalyse qui est nécessaire pour obtenir une conversion poussée en esters méthyliques.

La totalité du mélange recueilli après 72 heures de marche est évaporé totalement au "rotavapor" de façon à éliminer l'excès de méthanol puis il est ensuite débarrassé du glycérol formé au cours de la transestérification par une décantation statique à 50 °C.

Le mélange constitué d'esters méthyliques à environ 94,7 % de pureté est ensuite réintroduit dans le réacteur avec une quantité de méthanol équivalente en masse soit 50/50, dans les mêmes conditions opératoires soit : une température de 200 °C et une pression comprise entre 50 et 60 bar (entre 5 et 6 MPa) et des débits respectifs en esters et méthanol de 35 ml et 40 ml/heure.

Le mélange réactionnel est évaporé totalement, puis après décantation le glycérol formé est séparé et le mélange d'esters obtenu est analysé par GPC. Les résultats sont consignés dans le Tableau 1.

**Tableau 1**

| **Composition des esters fabriqués à partir d'huile de colza semi-raffinée.** | | | | | |
|---|---|---|---|---|---|
| Huile de colza I.A. = 0,5 max | TG | DG + stérols | MG | EMC + acides gras | I.A. |
| 1^{ère} étape de catalyse | 0,4 | 1,7 | 2,2 | 94,7 | 0.3 |
| 2^{ème} étape de catalyse | 0 | 0,75 | 0,5 | 98,75 | 0,2 |

| | | | | | |
|---|---|---|---|---|---|
| TG : Triglycérides (huile) DG : Diglycérides MG : Monoglycérides EMC : Esters méthyliques de colza I.A. : Indice d'Acide | | | | | |

### EXEMPLE 2

Le mode opératoire est le même que celui décrit dans l'Exemple 1, mais la nature de l'huile de colza utilisée diffère par son indice d'acide qui est cette fois égale à 11.

Cette huile a été reconstituée à partir d'un mélange pesé d'acide oléique distillé et d'huile de colza semi-raffinée, identique à celle utilisée dans l'Exemple 1.

Trois kilogrammes de cette huile ont été élaborés de la façon suivante : mélange de 165 g d'acide oléique distillé et 2835 g d'huile de colza semi-raffinée. L'indice d'Acide de ce mélange à été déterminé en utilisant la Norme NF ISO 660 et a donné un I.A = 11.

Deux étapes de catalyse ont été réalisées dans les mêmes conditions que l'Exemple 1.

Les résultats sont consignés dans le Tableau 2.

**Tableau 2**

| **Composition des esters fabriqués à partir d'huile acide** | | | | | |
|---|---|---|---|---|---|
| Huile de colza I.A. = 11 | TG | DG+ stérols | MG | EMC+ acides gras | I.A. |
| 1^{ère} étape de catalyse | 4,10 | 4,90 | 4,20 | 86,80 | 1,6 |
| 2^{ème} étape de catalyse | 0,05 | 0,95 | 0,60 | 98,40 | 0,35 |

| | | | | | |
|---|---|---|---|---|---|
| TG : Triglycérides (huile) DG : Diglycérides MG : Monoglycérides EMC : Esters méthyliques de colza I.A. : Indice d'Acide | | | | | |

L'eau produite au cours de la réaction d'estérification se retrouve piégée en majorité dans la phase glycérine. Cette eau est également un inhibiteur de la catalyse comme on peut en juger dans l'Exemple 3.

### EXEMPLE 3

On effectue une série d'essais (5 tests) concernant la première étape de catalyse, en faisant varier la teneur en eau de la charge et en suivant le même mode opératoire que dans l'Exemple 1.

L'huile de colza semi-raffinée a un indice d'acide I.A. = 0,5 max. et une teneur en eau de 400 ppm d'eau. Le méthanol contient successivement les quantités d'eau suivantes : 500, 1500, 3000 et 6000 ppm.

Le temps de marche de chaque test est de 48h pour assurer une bonne stabilité de la conversion. Un point retour est réalisé en fin de test utilisant du méthanol sec (500 ppm d'eau).

Le mélange huile méthanol étant de 50/50 en masse, les différentes teneurs en eau de la charge seront donc respectivement pour les 4 tests envisagés de 450, 950, 1750 et 3250 ppm.

Les résultats obtenus sont consignés dans le Tableau 3.

**Tableau 3**

| **Influence de la teneur en eau dans la charge (huile + méthanol) sur la composition des esters fabriqués** | | | | | | |
|---|---|---|---|---|---|---|
| Teneur en eau dans la charge (ppm) | | TG | DG + stérols | MG | EMC + acides gras | I.A. |
| Test 1 | 450 | 0,4 | 1,7 | 2,2 | 94,7 | 0,12 |
| Test 2 | 950 | 1,4 | 2,7 | 3,0 | 92,9 | 0,17 |
| Test 3 | 1750 | 3,3 | 3,8 | 4,8 | 88,1 | 0,30 |
| Test 4 | 3250 | 4,6 | 5,5 | 5,2 | 84,7 | 0,68 |
| Test 5 | 450 | 0,5 | 1,7 | 2,3 | 94,5 | 0,12 |

| | | | | | | |
|---|---|---|---|---|---|---|
| TG : Triglycérides (huile) DG : Diglycérides MG : Monoglycérides EMC : Esters méthyliques de colza I.A. : Indice d'Acide | | | | | | |

On remarque que la variation du taux de conversion en esters méthyliques est inversement proportionnelle à la teneur en eau contenue dans la charge (huile + méthanol).

L'eau joue un rôle d'inhibiteur, mais cette action est labile car en revenant avec le (test 5) aux conditions de marche initiales (test 1) avec 450 ppm d'eau dans la charge, on retrouve une conversion en esters méthyliques identique.

La réaction d'estérification génère de l'eau, donc la teneur en eau de la charge va augmenter et devenir critique pour la conversion en esters méthylique. On va donc se retrouver rapidement dans des conditions de fonctionnement se rapprochant de celles de l'Exemple 3 qui dépendent de l'indice d'acide de l'huile utilisée.

En analysant les résultats du Tableau 2 relatif à l'Exemple 1, où à partir d'une huile d'indice d'acide de 11, on obtient en fin de première catalyse un mélange dont l'indice d'acide est de 1,6, on peut par calcul connaître la quantité d'eau générée correspondant à l'estérification de l'équivalent d'acide gras. On obtient dans ce cas, de l'ordre de 3000 ppm d'eau formée dans le mélange réactionnel.

En comparant ce résultat à ceux de l'Exemple 3 (Tableau 3), on constate que l'on est assez proche des valeurs de conversions obtenues en ajoutant des quantités d'eau équivalentes (Tableau 3 - test 4).

Par cette approche, le procédé de l'invention fixe ses limites et c'est à l'homme de l'art de définir une valeur maximum d'acidité de façon à ne pas pénaliser la composition du mélange d'esters d'acide gras résultant des 2 étapes de catalyse.

Pour dépasser ces limites, la multiplication des étapes de catalyse serait une solution envisageable si leurs coûts opératoires demeurent compétitifs et ceci comparés aux opérations opératoires permettant de réduire l'acidité des huiles acides comme précédemment citées avec comme exemple, une estérification par le méthanol par catalyse homogène via des acides minéraux du type sulfurique ou chlorhydrique.

## Revendications

1. Procédé d'alcoolyse d'huiles acides non désacidifiées d'origine végétale ou animale dont l'indice d'acide est compris entre 0,5 et 20, avec des monoalcools de C1 à C5, dans lequel on réalise deux étapes de catalyse dans deux réacteurs fonctionnant avec un catalyseur hétérogène en lit fixe, la transestérification desdites huiles végétales ou animales possédant une acidité libre naturelle, et l'estérification de leur acidité libre étant réalisées simultanément.

2. Procédé selon la revendication 1 **caractérisé en ce qu'**il comprend :
- une étape (a) de réaction catalytique, dans laquelle on introduit simultanément, une quantité aliquote d'huile et de monoalcool dans ledit premier réacteur, préchauffé à une température comprise entre 180 et de 210°C et sous une pression opératoire comprise entre 4 à 6 MPa ;
- une étape (b) dans laquelle on soumet le mélange réactionnel en sortie du premier réacteur de catalyse à une évaporation totale ou partielle de l'excès de monoalcool, favorisant la séparation du glycérol formé, que l'on récupère ;
- une étape (c) dans laquelle on introduit le mélange d'ester dans le deuxième réacteur avec addition de l'équivalent massique de monoalcool ;
- une étape (d) dans laquelle on soumet le mélange issu de l'étape (c) à une évaporation totale de l'excès de monoalcool, puis l'élimination du glycérol résiduel formé.

3. Procédé selon la revendication 1 ou 2 **caractérisé en ce que** le catalyseur utilisé dans les étapes (a) et (c) comprend un oxyde mixte aluminate de zinc et répond à la formule ZnAl₂O₄, x ZnO, y Al₂O₃, où x et y représentent chacun un nombre compris entre 0 et 2.

4. Procédé selon la revendication 3 **caractérisé en ce que**, dans le catalyseur, l'aluminate de zinc est de type spinelle.

5. Procédé selon la revendication 1 à 4 **caractérisé en ce que** les deux réacteurs sont de tailles substantiellement identiques et l'étape (c) est effectuée dans les mêmes conditions de catalyse que la première étape de catalyse (a).

6. Procédé selon l'une des revendications 1 à 5 **caractérisé en ce que** l'huile de départ est choisie parmi les huiles brutes démucilaginées de colza, de soja et de tournesol, naturellement riches en acides gras.

7. Procédé selon l'une des revendications 1 à 6 **caractérisé en ce que** l'huile de départ est choisie parmi les huiles exotiques de palme, de palmiste et de coprah, naturellement riches en acides gras.

8. Procédé selon l'une des revendications 1 à 7 **caractérisé en ce que** l'on utilise une huile acide brute débarrassée de ses phospholipides et/ou mucilages.

9. Procédé selon la revendication 8 **caractérisé en ce que** l'indice d'acide est compris entre 1 et 15.

10. Procédé selon la revendication 8 **caractérisé en ce que** l'indice d'acide est compris entre 2 et 12.

11. Procédé selon l'une des revendications 9 et 10 **caractérisé en ce que** l'huile est issue de pression et/ou d'extraction et a subi une étape de démucilagination, de façon à obtenir une teneur résiduelle en phosphore inférieure à 10 ppm, suivie d'une étape de séchage permettant d'obtenir une teneur en eau résiduelle inférieure à 500 ppm.

12. Procédé selon l'une des revendications 1 à 11 **caractérisé en ce que** le monoalcool est le méthanol.

## Claims

1. A process for the alcoholysis of non deacidified acid oils of vegetable or animal origin with an acid number in the range 0.5 to 20, with C1 to C5 mono alcohols, in which is carried out two catalysis steps in two reactors functioning with a heterogeneous fixed bed catalyst, transesterification of said vegetable or animal oils with a natural free acidity and esterification of their free acidity being done simultaneously.

2. A process according to claim 1, **characterized in that** it comprises:
• a catalytic reaction step a) in which an aliquot quantity of oil and the monoalcohol are simultaneously introduced into said first reactor, pre-heated to a temperature in the range 180°C to 210°C and at an operating pressure in the range 4 to 6 MPa;
• a step b) in which the reaction mixture leaving the first catalysis reactor undergoes complete or partial evaporation of the excess monoalcohol, promoting separation of the glycerol formed, which is recovered;
• a step c) in which the ester mixture is introduced into the second reactor with addition of the equivalent by weight of monoalcohol;
• a step d) in which the mixture from step c) undergoes complete evaporation of the excess monoalcohol, then the elimination of the residual glycerol formed.

3. A process according to claim 1 or claim 2, **characterized in that** the catalyst used in steps a) and c) comprises a zinc aluminate mixed oxide and with formula ZnAl₂O₄, xZnO, yAl₂O₃, in which x and y each represent a number in the range 0 to 2.

4. A process according to claim 3, **characterized in that** the zinc aluminate of the catalyst is of the spinel type.

5. A process according to claims 1 to 4, **characterized in that** the two reactors are substantially identical in size and step c) is carried out under the catalysis conditions of the first catalysis step a).

6. A process according to one of claims 1 to 5, **characterized in that** the starting oil is selected from unrefined, naturally fatty acid-rich degummed rapeseed, soya and sunflower oil.

7. A process according to one of claims 1 to 6, **characterized in that** the starting oil is selected from naturally fatty acid-rich exotic African palm, palm nut oil and coconut oil.

8. A process according to one of claims 1 to 7, **characterized in that** an unrefined acid oil freed of its phospholipids and/or gums.

9. A process according to claim 8, **characterized in that** the acid number is between1 and 15.

10. A process according to claim 8, **characterized in that** the acid number is between 2 and 12.

11. A process according to claim 9 or claim 10, **characterized in that** the oil results from pressure and/or extraction and has undergone a degumming step to obtain a residual phosphorous content of less than 10 ppm followed by a drying step to obtain a residual water content of less than 500 ppm.

12. A process according to one of claims 1 to 11, **characterized in that** the mono alcohol is methanol.

## Patentansprüche

1. Verfahren zur Alkoholyse von nicht entsäuerten, sauren Ölen pflanzlichen oder tierischen Ursprungs, deren Säurezahl im Bereich zwischen 0,5 und 20 liegt, mit C1- bis C5-Monoalkoholen, bei dem zwei Katalyseschritte in zwei Reaktoren ausgeführt werden, die mit einem heterogenen Katalysator im Festbett arbeiten, wobei die Umesterung der pflanzlichen oder tierischen Öle, die eine natürliche freie Azidität besitzen, und die Veresterung ihrer freien Azidität gleichzeitig ausgeführt wird.

2. Verfahren nach Anspruch 1, **dadurch gekennzeichnet, dass** es umfasst:
- einen Schritt (a) zur katalytischen Reaktion, bei dem gleichzeitig eine aliquote Menge an Öl und Monoalkohol in den ersten Reaktor, der auf eine Temperatur im Bereich zwischen 180 und 210 °C vorerhitzt ist, und bei einem Betriebsdruck im Bereich zwischen 4 und 6 MPa eingeführt wird;
- einen Schritt (b), bei dem das Reaktionsgemisch am Ausgang des ersten Katalysereaktors einer vollständigen oder teilweisen Verdampfung des Monoalkoholüberschusses unterzogen wird, die die Trennung des gebildeten Glycerols, das zurückgewonnen wird, begünstigt;
- einen Schritt (c), bei dem das Estergemisch in den zweiten Reaktor unter Zugabe einer äquivalenten Masse an Monoalkohol eingeführt wird;
- einen Schritt (d), bei dem das Gemisch, das aus Schritt (c) stammt, einer vollständigen Verdampfung des Monoalkoholüberschusses, dann der Entfernung des gebildeten Restglycerols unterzogen wird.

3. Verfahren nach Anspruch 1 oder 2, **dadurch gekennzeichnet, dass** der in den Schritten (a) und (c) verwendete Katalysator ein Zinkaluminat-Mischoxid enthält und der Formel ZnAl₂O₄, xZnO, yAl₂O₃ entspricht, worin x und y jeweils für eine Zahl zwischen 0 und 2 stehen.

4. Verfahren nach Anspruch 3, **dadurch gekennzeichnet, dass** in dem Katalysator das Zinkaluminat vom Spinelltyp ist.

5. Verfahren nach Anspruch 1 bis 4, **dadurch gekennzeichnet, dass** die zwei Reaktoren im Wesentlichen die gleiche Größe aufweisen und dass Schritt (c) unter den gleichen Katalysebedingungen wie der erste Katalyseschritt (a) durchgeführt wird.

6. Verfahren nach einem der Ansprüche 1 bis 5, **dadurch gekennzeichnet, dass** das Ausgangsöl ausgewählt ist aus unraffinierten, entschleimten Raps-, Soja- und Sonnenblumenölen, die von Natur aus reich an Fettsäuren sind.

7. Verfahren nach einem der Ansprüche 1 bis 6, **dadurch gekennzeichnet, dass** das Ausgangsöl ausgewählt ist aus exotischen Palm-, Palmkern-und Kokosnussölen, die von Natur aus reich an Fettsäuren sind.

8. Verfahren nach einem der Ansprüche 1 bis 7, **dadurch gekennzeichnet, dass** ein unraffiniertes saures Öl verwendet wird, aus dem die Phospholipide und/oder die Schleime entfernt wurden.

9. Verfahren nach Anspruch 8, **dadurch gekennzeichnet, dass** die Säurezahl im Bereich zwischen 1 und 15 liegt.

10. Verfahren nach Anspruch 8, **dadurch gekennzeichnet, dass** die Säurezahl im Bereich zwischen 2 und 12 liegt.

11. Verfahren nach einem der Ansprüche 9 und 10, **dadurch gekennzeichnet, dass** das Öl aus Pressung und/oder Extraktion stammt und einem Schritt zur Entschleimung, um einen Restgehalt an Phosphor von weniger als 10 ppm zu erhalten, gefolgt von einem Schritt zur Trocknung, der es ermöglicht, einen Restgehalt an Wasser von weniger als 500 ppm zu erhalten, unterzogen wurde.

12. Verfahren nach einem der Ansprüche 1 bis 11, **dadurch gekennzeichnet, dass** der Monoalkohol Methanol ist.
